# EUROPEAN PATENT SPECIFICATION

(11) **EP 3 139 791 B1**
(45) Date of publication and mention of the grant of the patent: **01.01.2020**
(21) Application number: 15789029.4
(22) Date of filing: 08.05.2015
(51) Int. Cl.: A61K 8/34, A61K 8/37, A61K 8/92, A61K 8/97, A61Q 17/00, A61Q 19/00, A61Q 19/10, A61H 35/02, A61K 8/20, A61F 13/40, A61K 9/00

(54) **SYSTEMS, METHODS, AND KITS FOR CLEANSING AN OCULAR REGION**
SYSTEME, VERFAHREN UND KITS ZUR REINIGUNG EINER OKULAREN REGION
SYSTÈMES, PROCÉDÉS ET KITS DESTINÉS AU NETTOYAGE D'UNE RÉGION OCULAIRE

(30) Priority: 09.05.2014 US 201414274198
(43) Date of publication of application: 15.03.2017
(73) Proprietor: Ocusoft, Inc., Richmond, Texas 77406 (US)
(72) Inventor: SMITH, Troy, Richmond, TX 77406 (US)
(74) Representative: FRKelly
(86) International application number: PCT/US2015/029889
(87) International publication number: WO 2015/172022

(56) References cited:
- WO-A1-2013/156808
- US-A1- 2003 213 820
- US-A1- 2006 246 013
- US-A1- 2009 137 533
- US-A1- 2009 137 533
- US-A1- 2012 121 693
- US-A1- 2012 121 694
- US-A1- 2014 117 040
- US-A1- 2014 117 040
- US-B1- 6 726 386

## Description

### FIELD OF THE INVENTION

Systems, methods, and kits useful for cleansing the eyelids and maintaining eyelid hygiene are disclosed.

### BACKGROUND OF THE INVENTION

The eyelids are important to ocular health because they protect the eyes from airborne contaminants, such as pollen, dust particles or other foreign bodies. The eyelids contain several glands including the lacrimal glands and the specialized form of the sebaceous glands, the meibomian glands, which produce layers of tear film that are critical for healthy eyes.

The eyelids are subject to problems like blepharitis, dry eyes and inflammation of the meibomian glands. Another complication is the infestation of the arachnid, *Demodex* folliculorum (*Demodex* mites). The *Demodex* mite infestation is common in humans; anecdotal evidence suggests that the mites can be found in ten percent of the eyelashes of healthy persons. The occurrence of the infestation may also be age related.

*Demodex* mites reside inside the sebaceous glands and hair follicles. They cause damage to the cell walls by sucking nutrients from the hair roots. They burrow into the skin, lay eggs, introduce bacteria, and infect the skin. Some of the symptoms of infestation include itching and inflammation of the eyelids. Additionally, there is evidence that the mites can also be one of the causes of the skin disease rosacea.
US 2009/0137533 A1 discloses an eyelid treatment kit used for combination therapy for improving overall eyelid hygiene while also providing for adjunctive eyelid therapy. The eyelid treatment kit comprises low dose doxycycline hyclate tablets, a non-irritating eyelid cleansing composition, an anti-bacterial eyelid preparation and at least one pair of moist heat goggles and/or one pair of moisture chamber goggles. The eyelid treatment kit further comprises instruction sheets containing dosage and administration information on the doxycycline hyclate coupled with information on improving eyelid hygiene. The various embodiments of the eyelid treatment kit facilitate treatment of dry eyes due to infected eyelids, and cleansing of the eyelids to prevent recurring infections.
US 2014/0117040 A1 discloses a dispenser for dispensing flowable materials has a container assembly having a dividing wall. A first chamber and a second chamber are defined in the container assembly. The first chamber is configured to contain a first flowable material (Ml) and the second chamber is configured to contain a second flowable material (M2). A membrane is positioned in the container and cooperates with the dividing wall to enclose the first and second chambers. The membrane has a first section having a first weld seam and a second section having a second weld seam. The first section is separated from the second section by a non-rupturable member. Rupture of the membrane allows the flowable materials into a third chamber in which the flowable materials mix to form a mixture, and from which the mixture is dispensed.

### SUMMARY OF THE INVENTION

Systems, methods, and kits useful for cleansing the eyelids and maintaining eyelid hygiene are disclosed. In one embodiment, a system for treating or cleansing an ocular region is disclosed. The system is defined in the claims below and consists essentially of: (A) a tubular applicator having a first end and an opposed second end, the first end having an opening, wherein the tubular applicator consists of: (i) a first chamber and (ii) a second chamber, wherein the first chamber is adjacent the first end of the applicator and the second chamber is adjacent the second end of the of the applicator; (iii) a rupturable sealable element situated between the first chamber and the second chamber and disposed within an internal cavity of the tubular applicator, the sealable element defining the first chamber and the second chamber within the internal cavity, and (iv) a self-saturating dispenser for the ocular composition, wherein the dispenser is bonded to an external surface of a first end of the applicator, wherein the dispenser envelopes the opening at the first end of the applicator, wherein the dispenser comprises an absorbent material selected from the group consisting of foam, sponge, fiber, felt, cotton, rayon, synthetic foam, synthetic sponge, textile and synthetic fiber; and (B) an ocular composition; wherein the second chamber is pre-filled with the ocular composition, wherein rupturing of the sealable element creates a passage between the first and second chambers for the ophthalmic composition to flow from the second chamber to the first chamber and through the opening at the first end, wherein the dispenser is configured to receive a desired amount of the ocular composition through the opening.

Also disclosed is a method of treating or cleansing an ocular region. The method includes (i) providing the system for treating or cleansing an ocular region; (ii) substantially filling at least the second chamber of the applicator with the ocular composition; and (iii) causing the sealable element to break such that the ocular composition is capable of flowing from the second chamber to the first chamber.

Also disclosed is a kit for treating or cleansing an ocular region. The kit includes an enclosure for one or more of the systems for treating or cleansing an ocular region. Each of the systems may be individually wrapped.

Also disclosed is a kit for debridement of Anterior Blepharitis comprises an enclosure for housing: (i) one or more individually wrapped packages containing the system described above, wherein the ocular composition comprises polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate; and (ii) a double-ended soft brush for removing debris from the eyelids and eyelashes.

Also disclosed is a method for treating Anterior Blepharitis includes: (i) providing the kit for debridement of Anterior Blepharitis; (ii) holding the applicator with the dispenser facing downward; (iii) applying pressure on a tip of the applicator that is opposite the dispenser to rupture the sealable element thereby enabling the ocular composition to saturate the dispenser; (iv) cleansing the surface of one or more of the eyebrows, closed eyelids and eyelashes by dispensing the ocular composition through the dispenser; (v) allowing the ocular composition to remain on the cleansed surface; and (vi) using the brush to remove any debris from the cleansed surface.

In another embodiment, a kit for debridement of Demodex includes an enclosure for housing: (i) at least a first individually wrapped package containing the system described in claim 1, wherein the ocular composition comprises a first ocular composition, the first ocular composition comprising polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate; (ii) at least a second individually wrapped package containing the system described in claim 1, wherein the ocular composition comprises a second ocular composition, the second ocular composition comprising a mixture of tea tree oil, sea buckthorn oil and a medium chain triglyceride; (iii) at least one of a double-ended soft brush or a pair of tweezers for removing debris from the eyelids and eyelashes; and (iv) one or more sterile saline wipes.

In another embodiment, an ocular composition for use in a method for treating Demodex is provided, the method including: (i) providing the kit for treating Demodex as described claim 7; (ii) holding the first individually wrapped package such that the dispenser end is pointed downward and pressing firmly on a tip of the applicator that is opposite the dispenser to rupture the sealable element thereby enabling the first ocular composition to saturate the dispenser; (iii) cleansing a surface of the eyebrows, closed eyelids and eyelashes by dispensing the first ocular composition through the dispenser; (iv) removing any debris from the area to be treated using either the brush or tweezers; (v) removing the first ocular composition by rinsing the cleansed surface; (vi) holding the second individually wrapped package such that the dispenser end is pointed downward and pressing firmly on a tip of the applicator that is opposite the dispenser to rupture the sealable element thereby enabling the second ocular composition to saturate the dispenser; and (vii) treating the cleansed surface by gently applying the second ocular composition to the cleansed surface. The method may further comprise removing any additional debris from treated surface and further involves removing the second ocular composition. In another embodiment, the ocular composition for use in a method for treating Demodex, further comprises repeating steps (i) - (iv), wherein the first ocular composition is allowed to remain on the cleansed surface.

In another embodiment, a kit for meibomian gland expression includes an enclosure for housing: (i) the system described in claim 1, wherein the ocular composition comprises polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate; (ii) a pair of moist heat therapy goggles for combining moisture and heat to open up clogged meibomian glands by stimulating tear production and preventing evaporation of tears; (iii) one or more anesthetic gels; (iv) a paddle for expressing meibum from the meibomian glands; (v) a double-ended brush for removing debris from the eyelids and eyelashes; (vi) an eye wash for irrigating or flushing foreign bodies and debris from the eye; (vii) an eyelid cleanser for removing oil, debris and pollen from the eyelids; (viii) an eyelid spray containing vitamins A, C and E; and (ix) an ophthalmic emulsion to stabilize tear film and protect against moisture loss.

In another embodiment, an ocular composition for use a method for meibomian gland expression is provided, the method including: (i) providing the kit for meibomian gland expression as described in claim 12; (ii) instructing a patient to wear the moist heat therapy goggles for at least around 10- 15 minutes in order to open up the clogged meibomian glands; (iii) applying the anesthetic gel to the eyes; (iv) after about 4-10 minutes, expressing the meibomian glands with the paddle; (v) cleansing closed eyelids to remove excessive sebum/oil from the eyelids by dispensing the ocular composition through the dispenser; (vi) removing debris from the cleansed eyelids using the brush; and (vii) irrigating out the eye using the eye wash.

### Brief Description of the Figures

The features and advantages of certain embodiments will be more readily appreciated when considered in conjunction with the accompanying figures. The figures are not to be construed as limiting any of the preferred embodiments.
Fig. 1 depicts a system for treating or cleansing an ocular region according to one embodiment of the invention.
Figs. 2A and 2B depict kits for treating or cleansing an ocular region according to one embodiment of the invention.

### DETAILED DESCRIPTION

As used herein, an ocular region includes the eyes, eyelids, periorbital region or an area adjacent the eyes, eyelids and periorbital region. The periorbital region includes or relates to the tissues surrounding or lining the orbit of the eye. The orbit of the eye is the cavity of the skull in which the eye and related nerves and blood vessels are located.

Several compositions for cleansing or treating the ocular region are known in the art. These treatment compositions are usually applied to the ocular region by means of the finger tips, dispensers or applicators. Applicators can include swabs, Q-tips®, sponges, etc. In some instances, the fabric pads may be pre-soaked or pre-treated with a desired amount of treatment composition.

However, it has been observed that even these pre-treated applicators may not retain an optimal amount of the treatment composition. Seepage of the composition into the package(s) may cause a loss in the amount of the treatment composition retained in/by the applicator. The applicators or pre-treated fabric pads may also be inconvenient or messy to handle. For example, the treatment composition may be greasy, or drip from the applicator during use and may get on the fingers or clothes. Consequently, the overall efficacy of the treatment may be impacted.

Accordingly, for all the reasons stated above, there is a need for a mechanism to deliver a desired amount of an ocular composition to an ocular region. The mechanism should be convenient to use at home or in a doctor's office. The mechanism should facilitate a non-messy treatment of the ocular region.

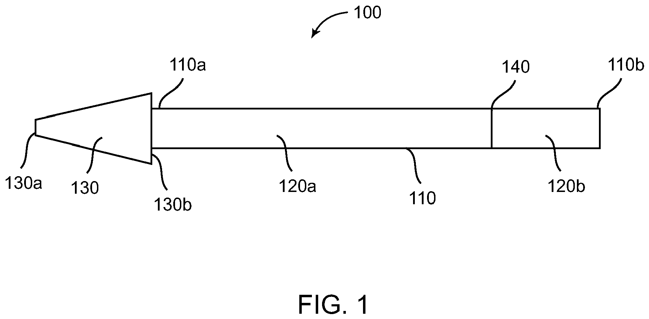

In one embodiment, a system suitable for both adjunctive eyelid therapy and hygiene maintenance is provided. Referring now to Figure 1, in one embodiment of the invention, a system for cleansing or treating an ocular region 100 consists essentially of a tubular applicator 110 comprising a first chamber 120a and a second chamber 120b. A sealable element 140 is positioned between the first chamber 120a and the second chamber 120b. At least the second chamber 120b is pre-filled with an ocular composition. A dispenser 130 isbonded to an external surface of a first end of the applicator 110a.

The applicator 110 includes a tubular enclosure or container. The applicator 110 may be manufactured of a suitable material known in the art. For example, the material may include plastic, polymer, resin, synthetic polymer and a composite material. In one embodiment, the applicator 110 may comprise a substantially clear polypropylene tube. Polypropylene is suitable and desirable material since it is lightweight, flexible and does not shatter. Polypropylene is also free of undesirable chemicals, such as, BPA. The applicator 110 may be dimensioned for convenient handling and such that it can hold a desired amount of the ocular composition. For example, the applicator 110 may have an external diameter that ranges from about 4 mm to about 8 mm and its length (that is, the distance between the first end 110a and the second end 110b) may range from about 75 mm to about 95 mm.

The applicator 110 includes an internal cavity that ise divided into the first chamber 120a and the second chamber 120b by an internal sealable element 140. As used herein, the term "chamber" is intended to encompass a compartment, enclosure or closed space. The sealable element 140 may be located substantially adjacent the second end of the applicator 110b. However, the sealable element 140 may also be located anywhere inside the applicator 110. For example, the sealable element 140 may be located substantially in the middle portion (not shown) of the applicator 110 or the sealable element 140 may be located substantially adjacent the first end of the applicator 110a (not shown). Accordingly, the first chamber 120a and the second chamber 120b may or may not be uniform in size. The sealable element 140 includes any mechanism known in the art that can be ruptured, either completely or partially, by the application of pressure or an external force. For example, the sealable element 140 may include a membrane that can be broken by pressing the end 110b firmly.

A dispenser 130 is bonded to the first end of the applicator 110a. As used herein, the term "dispenser" is intended to include an object that dispenses or that allows the distribution of a desired amount of the ocular composition. The dispenser 130 is made of an absorbent material that is self-saturating or pervious to the ocular composition and is also capable of retaining a desired amount of the ocular composition. The dispenser 130 includes an absorbent material such as, foam, sponge, fiber, felt, cotton, rayon, synthetic foam, synthetic sponge, textile and synthetic fiber. In one embodiment, the dispenser 130 may be rectangular shaped and may be made of 100PPI medical grade foam. The dispenser 130 may be dimensioned to receive a desired amount of the ocular composition through an opening (not shown) at the first end of the applicator 110a. In one embodiment, the dispenser 130 may range from about 15 mm to about 20 mm in diameter and its length (that is, the distance between the first end of the dispenser 130a and the second end of the dispenser 130b) may range from about 30 mm to about 40 mm. The dispenser 130 envelopes the opening at the first end of the applicator 110a.

The ocular composition can be in a form chosen from an emulsion, a suspension, a dispersion, a foam, a cream, a lotion, a solution, a paste, a gel or a spray. The ocular composition may be capable of cleansing the ocular region or treating the ocular region. The ocular composition may be a non-irritating liquid composition useful in cleansing the eyelids. The ocular composition may also be effective as an eyelid cleanser, as it has an antimicrobial effect. The use of non-irritating ingredients that also exhibit antimicrobial benefits in the ocular composition increases its cleansing ability. The ocular compositions may offer convenient combination therapy for improving overall eyelid hygiene and also providing for adjunctive eyelid therapy.

For example, the ocular composition may include a surfactant mixture comprising PEG-80 sorbitan laurate, sodium trideceth sulphate, PEG-150 distearate, cocamidopropylhydroxy sultaine, lauroamphocarboxy glycinate, and sodium laureth-13 carboxylate, the surfactant mixture present in a concentration of 7-10%; PEG-15 tallow polyamine present in a concentration of 0.1-0.5%; sodium chloride present in a concentration of 0.6-0.9%, at least one microbiological preservative selected from the group consisting of Quaternium-15 and benzyl alcohol present in concentration of 0.1-0.5% and a chelating agent present in a concentration of 0-0.1%.

In another embodiment, the ocular composition may include the eyelid scrub composition disclosed in U.S. Pat. No. 7,951, 387 assigned to OCuSOFT, Inc. For example, the ocular composition comprises PHMB, 1,2-hexanediol, and 1,2-octanediol in combination with a pH stabilizing surfactant solution. Suitable surfactants to be used in the pH stabilizing surfactant solution include amphoteric surfactants, anionic surfactants, and nonionic surfactants. Suitable amphoteric surfactants include, but are not limited to alkyldimethyl betaines, alkylamido betaines, sulfobetaines, and imidazoline amphoterics. Suitable anionic surfactants include, but are not limited to fatty alcohol sulfates, alpha olein sulfonates, sulfosuccinates, sarcosinates, phosphate esters, and carboxylates. Suitable nonionic surfactants include, but are not limited to alkanolamids, ethoxylate amids, esters, aixylated alcohols, alkylpolyglucosides, amine oxides, sorbitan esters, and ethoxylates. The pH stabilizing surfactant solution comprises cocoamphodiacetate disodium, polyoxyethylene 80 sorbitan monolaurate, decyl polyglucoside, and a modified Ringer's solution. Cocoamphodiacetate disodium is an amphoteric surfactant. Polyoxyethylene 80 sorbitan monolaurate and decyl polyglucoside are both nonionic surfactants. In another embodiment, the composition, when mixed, comprises about 0.1 to 25 wt. % cocoamphodiacetate disodium, 0.1 to 10 wt. % polyoxyethylene 80 sorbitan monolaurate, 0.2 to 10 wt. % decyl polyglucoside, and 60 to 98 wt. % modified Ringer's solution. The modified Ringer's solution comprises, sodium chloride, potassium chloride, calcium chloride, and water. Preferably, the water used is purified water. The modified Ringer's solution may also comprise 0.05 to 1.2 wt. % sodium chloride, 0.005 to 0.5 wt. % potassium chloride, 0.005 to 0.5 wt. % calcium chloride, and water. In still another embodiment, the modified Ringer's solution comprises about 0.7 wt. % sodium chloride, about 0.03 wt. % potassium chloride, about 0.033 wt. % calcium chloride, and purified water.

The ocular composition can further comprise one or more moisturizers. Moisturizers are chemicals that prevent transepidermal water loss. Moisturizers may prevent water loss by forming a film over the skin to prevent water from evaporating from the skin. Alternatively, moisturizers comprise hydroscopic molecules that draw water from the air into the skin. Suitable moisturizers include, but are not limited to, methyl gluceth-20, sorbital, glycerine, propylene glycol, carboxylates, amino acids, glucoside derivatives, urea, lactates, and derivatives of pantothenic acid. Examples of derivatives of pantothenic acid include panthenol, D-panthenol, and D, L-panthenol.

The ocular composition may also include a foam stabilizer. A foam stabilizer is a chemical which increases the lifetime of the foam. The foam stabilizer can be a polyethylene glycol diester of methyl glucose and a fatty acid. Suitable fatty acids include oleic acid, steric acid, lauric acid, caprylic acid, and capric acid. Suitably, the foam stabilizer is PEG-120 methyl glucose dioleate.

One specific embodiment of the ocular composition comprises polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate.

Another specific embodiment of the ocular composition comprises about 0.02 wt. % to about 0.3 wt. % PHMB, about 0.05 wt. % to about 2.0 wt. % 1,2-hexanediol, about 0.05 wt. % to about 2.0 wt. % 1,2-octanediol, about 0.1 wt. % to about 25 wt. % cocoamphodiacetate disodium, about 0.1 wt. % to about 10 wt. % polyoxyethylene 80 sorbitan monolaurate, about 0.2 wt. % to about 10 wt. % decyl polyglucoside, and about 60 wt. % to about 98 wt. % Modified Ringer's Solution.

Yet another specific embodiment of the ocular composition comprises about 0.04 wt. % polyhexamethylene biguanide, about 0.2 wt. % 1,2-hexanediol, about 0.2 wt. % 1,2-octanediol, about 0.2 wt. % D-panthenol, about 0.215 wt. % cocoamphodiacetate disodium, about 4.032 wt. % polyoxyethylene 80 sorbitan monolaurate, about 0.275 wt. % decyl polyglucoside, about 4.3 wt. % methyl gluceth-20, about 0.6 wt. % PEG-120 methyl glucose dioleate, about 87.985 wt. % Modified Ringer's Solution, and water.

Zinc salts are astringents which cause skin to tighten. The skin around the ocular area is more sensitive that other areas of skin. The inclusion of a zinc salt is a composition may be undesirable as its astringent property would make the composition more irritating to the eyelid area. Therefore in one embodiment, the ocular composition comprises PHMB, 1,2-hexanediol, 1,2-octanediol, and a pH stabilizing surfactant solution, but is also essentially free of zinc salts. Examples of zinc salts include zinc acetate, zinc lactate, zinc gluconate, zinc citrate, zinc butyrate, and zinc sterate.

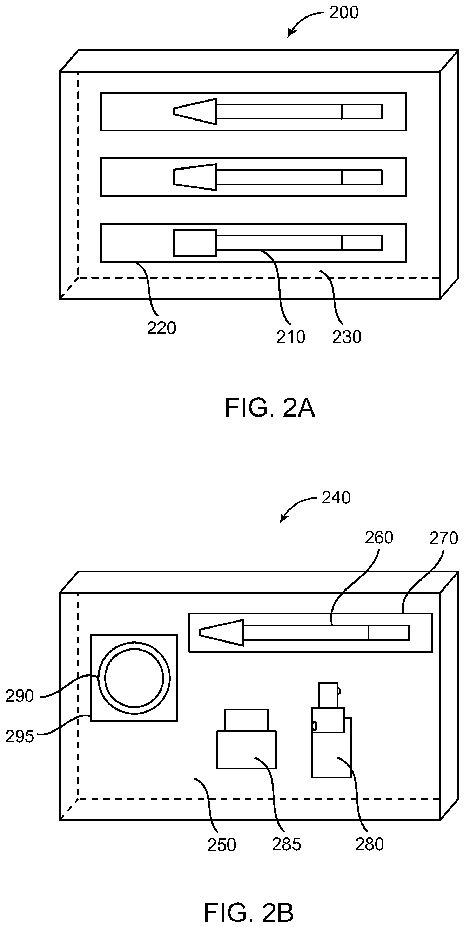

A kit 200 for debridement of Anterior Blepharitis is also disclosed. As shown in Fig.2A, the kit 200 may include an enclosure or housing 230. The enclosure 230 may be made of paper, plastic or another suitable material known in the art. One or more systems 210 for treating an ocular region (100) described earlier may be packaged within the enclosure 230. Each of the systems 230 may be further individually wrapped 210. The individually wrapped 210 packaging may further include an ocular composition comprising polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate. The kit 200 for debridement of Anterior Blepharitis may further include a specialized double-ended soft brush (not shown) for removing scurf and debris from the eyelids and eyelashes.

A method for treating a patient having Anterior Blepharitis using the above-described kit for debridement of Anterior Blepharitis is also disclosed. The patient can be treated in a doctor's office, such as, an ophthalmologist's office. The method involves the following steps: 1) providing one or more individually wrapped packages containing the system described herein, wherein the system comprises a combination of an applicator and an composition comprising polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate; 2) with the system still contained within the packaging, holding the applicator with the dispenser facing downward; 3) with the system still contained within the packaging, pressing firmly on the tip of the applicator at the end that is opposite the dispenser to rupture the sealable element to enable the ocular composition to saturate the dispenser; 4) removing the applicator from the packaging; 5) using the applicator as an handle, cleansing an area to be treated, for instance, the eyebrows, closed eyelids and eyelashes by dispensing the ocular composition through the dispenser; 6) allowing the ocular composition to remain on the area to be treated (that is, the area to be treated is not rinsed); and 7) removing any scurf or debris from the area to be treated. For example, a specialized brush can be used to remove the scurf or debris.

A method for ongoing maintenance of an area treated for Anterior Blepharitis involves the following steps: 1) cleansing the area treated in the doctor's office daily with an ocular composition dispensed in pre-moistened pads or as a cleanser (for example, OCuSOFT® Lid Scrub® PLUS pre-moistened pads or Foaming Eyelid Cleanser), for at least the first two weeks post-treatment in the doctor's office; 2) using the ocular composition daily for routine eyelid hygiene; 3) applying an eyelid spray (for example, Tears Again® ADVANCED Eyelid Spray) throughout the day, as needed, to soothe red, irritated eyelids; and 4) stabilizing the lipid layer of the tear film of the eye by applying an ophthalmic emulsion (for example, Retaine MGD® ophthalmic emulsion). The method further involves scheduling a follow-up appointment with the doctor for a second treatment, if required.

In yet another embodiment, the ocular composition may include a mixture of tea tree oil, sea buckthorn oil and a medium chain triglyceride. Such a composition has been found effective in treating Demodex mites. The composition has been disclosed in U.S. Pat. Pub. No. 20120121694. The medium chain triglyceride may comprise caprylic capric triglyceride or any other suitable dermatological carrier for the tea tree oil and sea buckthorn oil. For example, in one or more embodiments, the ocular composition may comprise about 45% to about 55% tea tree oil, about 15% to about 25% sea buckthorn oil, and about 25% to about 35% caprylic capric triglyceride. In another embodiment, the ocular composition may include 50% tea tree oil, 20% sea buckthorn oil, and 30% caprylic capric triglyceride.

In another embodiment of the invention, a kit 200 for debridement of Demodex is disclosed. As shown in Fig.2A, the kit 200 includes an enclosure or housing 230. The enclosure 230 may be made of paper, plastic or another suitable material known in the art. One or more systems 210 for treating an ocular region (100) described as described in claim 1 is packaged within the enclosure 230. Each of the systems 230 may be further individually wrapped 210. At least one of the individually wrapped 210 packages further includes a first ocular composition comprising polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate. At least one of the individually wrapped 210 packages my includes a second ocular composition comprising a mixture of tea tree oil, sea buckthorn oil and a medium chain triglyceride.

The kit 200 for debridement of Demodex further includes a specialized double-ended soft brush (not shown) for removing scurf and debris from the eyelids and eyelashes.

The kit 200 for treating Demodex may further include a pair of tweezers (not shown) for removing scurf and debris from the eyelids and eyelashes. The tweezers may have a cone tip and may be manufactured from plastic.

The kit 200 for debridement of Demodex further includes one or more sterile saline wipes. The wipes may be individually wrapped to provide safe and gentle cleansing of the eye area while eliminating the risk of cross-contamination.

In another embodiment, an ocular composition for use in a method for treating a patient having Demodex using the above-described kit for treatment of Demodex is disclosed. The patient can be treated in a doctor's office, such as, an ophthalmologist's office. The method involves the following steps: 1) providing the kit of claim 7 including at least one individually wrapped package containing the system described herein, wherein the system comprises a combination of an applicator and a first ocular composition comprising polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate; 2) with the system still contained within the packaging, holding the applicator with the dispenser facing downward; 3) with the system still contained within the packaging, pressing firmly on the tip of the applicator at the end that is opposite the dispenser to rupture the sealable element to enable the first ocular composition to saturate the dispenser; 4) removing the applicator from the packaging; 5) using the applicator as an handle, cleansing an area to be treated, for instance, the eyebrows, closed eyelids and eyelashes by dispensing the first ocular composition through the dispenser; 6) removing any debris from the area to be treated (for instance, a specialized brush or tweezers can be used); 7) removing the first ocular composition by rinsing the eye area (for instance, with OCuSOFT® Eye wash or the sterile saline wipes); 8) providing at least one individually wrapped package containing the system described herein, wherein the system comprises a combination of an applicator and a second ocular composition comprising a mixture of tea tree oil, sea buckthorn oil and a medium chain triglyceride; 9) with the system still contained within the packaging, holding the applicator with the dispenser facing downward; 10) with the system still contained within the packaging, pressing firmly on the tip of the applicator at the end that is opposite the dispenser to rupture the sealable element to enable the second ocular composition to saturate the dispenser; 11) removing the applicator from the packaging; 12) using the applicator as an handle, gently applying the second ocular composition to an area to be treated, for instance, the eyebrows, closed eyelids and eyelashes with the dispenser end of the applicator taking special care to avoid direct contact with the eye; 13) removing any additional debris from the area to be treated (for instance, a specialized brush or tweezers can be used); 14) removing the second ocular composition by rinsing the eye area (for instance, with OCuSOFT® Eye wash or the sterile saline wipes). In another embodiment, after step 14, steps 1-6 can be repeated and the first ocular composition can be allowed to remain on the area to be treated.

A method for ongoing maintenance of an area treated for Demodex involves the following steps: 1) cleansing the area treated in the doctor's office daily with the first ocular composition dispensed in pre-moistened pads or as a cleanser (for example, OCuSOFT® Lid Scrub® PLUS pre-moistened pads or Foaming Eyelid Cleanser), for at least the first two weeks post-treatment in the doctor's office; 2) using a third ocular composition (for example, OCuSOFT® Lid Scrub® Original pre-moistened pads or Foaming Eyelid Cleanser) daily for routine eyelid hygiene; 3) stabilizing the lipid layer of the tear film of the eye by applying an ophthalmic emulsion (for example, Retaine® MGD® ophthalmic emulsion); and 4) applying an ointment (for instance, Retaine® PM® Nighttime Ointment) for added night time relied. The method further involves scheduling a follow-up appointment with the doctor for a second treatment, if required.

The ocular composition consists essentially of purified water, PEG-80 sorbitan laurate, sodium trideceth sulfate, PEG-150 distearate, sodium lauroamphoacetate, cocamidopropyl hydroxysultaine, sodium laureth-13 carboxylate, sodium chloride, PEG-15 cocopolyamine, polyhexamethylene biguanide, potassium sorbate, 1,2 hexanediol, and caprylyl glycol.

Although various exemplary ocular compositions have been described herein, the compositions disclosed herein are not intended to be limiting. In fact, any composition that can be used to cleanse or treat the eyes, eyelids, or an ocular region, may be used in accordance with the various embodiments of the invention.

As described earlier, the sealable element 140 may be ruptured by the application of pressure. For example, the sealable element 140 may be conveniently snapped by a user of the system 100. Upon breaking the sealable element 140, a substantially contiguous opening is created between the first chamber 120a and the second chamber 120b in the applicator 110. This allows a passage for the ocular composition from the second chamber 120b to the first chamber 120a.

The first end 110a of the applicator 110 further includes an opening (not shown). The breaking of the sealable element 140 allows the ocular composition to flow through the opening such that it can be dispensed from the dispenser 130. Although not part of the claimed invention, the tubular applicator may not include a sealable element. The ocular composition is contained within the undivided chamber of the applicator (not shown). Upon pressing the tip of the tubular applicator that is at an opposing end to the dispenser, the ocular composition can be seamlessly pushed into the dispenser where it can saturate the absorbent material of the dispenser and thereby be dispensed to an area that requires cleansing or treatment.

A method of treating or cleansing an ocular region is also disclosed. The method includes the steps of providing the system (described earlier) for treating or cleansing an ocular region; (ii) substantially filling at least the second chamber of the applicator with the ocular composition; and (iii) causing the sealable element to break such that the ocular composition is capable of flowing from the second chamber to the first chamber. The method may further involve pre- or post-cleansing of the treatment area and follow-up treatment of the treatment area.

A kit 240 for treating or cleansing an ocular region is also disclosed. The kit 240 may include an enclosure or housing 250. The enclosure 250 may be made of paper, plastic or another suitable material known in the art. One or more systems 260 for treating an ocular region (100, 210) described earlier may be packaged within the enclosure 250. Each of the systems 260 may be further individually wrapped 270. The kit 240 may further include at least a sealable container 295 enclosing one or more fabric pads 290. The fabric pad 290 must be selected so that the fabric is capable of containing the ocular composition in the interstitial spaces of the fabric's weave. In one aspect, the fabric pad 290 comprises two sheets of fabric, a first sheet of fabric and a second sheet of fabric. The two pieces may be held together by stitching them together on the sides. The fabric pad 290 can have a surface area dimensioned to receive an effective amount of the ocular composition. In one aspect, the pre-moistened fabric pad 290 comprises a lint-free non-abrasive rayon and polypropylene fabric blend. In another aspect, the fabric pad 290 comprises a textured surface to absorb and retain the ocular composition. However, the fabric pad 290 must remain soft enough so as to not be harsh on the user's skin. Preferably, the fabric pads 290 further comprise a moisturizer blend that is non-drying and non-irritating.

The individually wrapped packages or sealable container 295 may be made of any suitable material including paper, plastic or a metal foil material. The pre-moistened fabric pads 290 may be individually packaged for convenience. In one aspect, the sealable container 295 may be an impervious wrapper so that the fabric pad 290 with the ocular composition does not come into contact with contaminants and remains moistened for a long period of time. The pre-moistened fabric pads 290 are applied to the eyelids or scrubbed using lateral side to side strokes on to the eyelids and other perioocular regions that need to be cleansed or treated. The eyelids are rinsed with water and the used fabric pads 290 are discarded.

The moisture pads 290 may be soaked in an ocular cleansing solution for convenience in cleansing the eyelids. The kit 240 may further include a first receptacle 285 containing an ocular composition and a second receptacle 280 containing a foaming ocular composition. The first and second receptacles 285, 280 may be selected from a glass bottle, a plastic bottle, or other suitable material known in the art. For convenience and economy, the receptacles 285, 280 may range in size from about 30 ml to 480 ml.

The kits 200, 240 may further include instructions. The instructions can be printed in a manual included in the kits 200, 240 on instruction sheets or they may be printed directly on the enclosures 230, 250. If the instructions are printing on the enclosures 230, 250, they may be printed on the outside of the enclosures 230, 250 or on the inside of the enclosures 230, 250 where the instructions are not visible to the user of the kits 200, 240 until the user opens the kits 200, 240. As an alternative, the instructions may be printed on the containers or packaging of the individual components of the eyelid treatment kit, such as, 220.

In yet another embodiment, a kit for meibomian gland expression (or Posterior Blepharitis) is provided. As shown in Fig.2A, the kit 200 includes an enclosure or housing 230. The enclosure 230 may be made of paper, plastic or another suitable material known in the art. One or more systems 210 for treating an ocular region (100) described in claim 1 is packaged within the enclosure 230. Each of the systems 230 may be further individually wrapped 210. The individually wrapped 210 packaging may further include an ocular composition comprising polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate.

The meibomian gland expression kit 200 further includes a pair of moist heat therapy goggles (not shown). The goggles can combine moisture and heat to open the clogged meibomian glands that contribute to inflammation and Dry Eye discomfort. The goggles can provide a comfort seal around the eyes to help stimulate tear production and prevent the evaporation of tears.

The meibomian gland expression kit 200 further includes one or more anesthetic gels (not shown). The gels may have a high viscosity to improve patient comfort and increase efficiency in the operating room or the clinic.

The meibomian gland expression kit 200 further includes a paddle to gently and effectively express meibum from the meibomian glands. When the paddle device is positioned behind the anesthetized eyelid parallel to the glands, the application of gentle digital pressure on the outer lid prompts the meibum to egress.

The meibomian gland expression kit 200 further includes a specialized double-ended brush (not shown) for removing scurf and debris from the eyelids and eyelashes.

The meibomian gland expression kit 200 further includes an eye wash (not shown) for irrigating or flushing foreign bodies and debris from the eye. The eyewash may include a purified water and sodium chloride ophthalmic irrigating solution, such as, OCuSOFT® Eye Wash.

The meibomian gland expression kit 200 further includes an eyelid cleanser (not shown) for removing oil, debris and pollen from the eyelids (not shown). The eyelid cleansers may comprise pre-moistened pads or as foaming cleansers.

The meibomian gland expression kit 200 further includes an eyelid spray. The eyelid spray may include liposomes containing vitamins A, C and E to soothe eyelid irritation while improving tear break up time (TBUT) and tear film stability.

The meibomian gland expression kit 200 further includes an ophthalmic emulsion. The ophthalmic emulsion can include a lipid-replenishing formula that utilizes electrostatic attraction to stabilize tear film and protect against moisture loss.

In yet another embodiment, an ocular composition for use in a method for meibomian gland expression using the meibomian gland expression kit described in claim 12 is disclosed. The patient can be treated in a doctor's office, such as, an ophthalmologist's office. The method involves the following steps: 1) instructing the patient to wear the moist heat therapy goggles for at least around 10- 15 minutes in order to open up the clogged meibomian glands; 2) anesthetizing the eyes with the anesthetic gel; 3) after waiting for around 4-10 minutes, expressing the meibomian glands with the paddle devices; 4) providing one or more individually wrapped packages containing the system described herein, wherein the system comprises a combination of an applicator and an composition comprising polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate; 5) with the system still contained within the packaging, holding the applicator with the dispenser facing downward; 6) with the system still contained within the packaging, pressing firmly on the tip of the applicator at the end that is opposite the dispenser to rupture the sealable element to enable the ocular composition to saturate the dispenser; 7) removing the applicator from the packaging; 8) using the applicator as an handle, cleansing an area to be treated, for instance, the closed eyelids to remove excessive sebum/oil from the eyelids by dispensing the ocular composition through the dispenser; 9) removing any scurf or debris from the area to be treated using the specialized brush; and 10) irrigating out the eye using the eye wash.

A method for ongoing maintenance of an area treated for meibomian gland expression involves the following steps: 1) using the moist heat therapy goggles daily to open the meibomian glands; 2) cleansing the area treated in the doctor's office daily with an ocular composition dispensed in pre-moistened pads or as a cleanser (for example, OCuSOFT® Lid Scrub® PLUS pre-moistened pads or Foaming Eyelid Cleanser), for at least the first two weeks post-treatment; 3) using the ocular composition daily for routine eyelid hygiene; 4) using a third ocular composition (for example, OCuSOFT® Lid Scrub® Original pre-moistened pads or Foaming Eyelid Cleanser) daily for routine eyelid hygiene; 5) applying an eyelid spray (for example, Tears Again® ADVANCED Eyelid Spray) throughout the day, as needed, to soothe red, irritated eyelids; and 6) stabilizing the lipid layer of the tear film of the eye by applying an ophthalmic emulsion (for example, Retaine® MGD® ophthalmic emulsion). The method further involves scheduling a follow-up appointment with the doctor for a second treatment, if required.

It should be understood that, as used herein, "first," "second," *etc*., are arbitrarily assigned and are merely intended to differentiate between two or more compartments, compositions *etc*., and does not indicate any particular orientation or sequence. Furthermore, it is to be understood that the mere use of the term "first" does not require that there be any "second," and the mere use of the term "second" does not require that there be any "third," *etc.*

Therefore, the present invention is well adapted to attain the ends and advantages mentioned as well as those that are inherent therein. The particular embodiments disclosed above are illustrative only, as the present invention may be modified and practiced in different but equivalent manners apparent to those skilled in the art having the benefit of the teachings herein and within the scope of the invention which is defined by the claims below. Furthermore, no limitations are intended to the details of construction or design herein shown, other than as described in the claims below. It is, therefore, evident that the particular illustrative embodiments disclosed above may be altered or modified and all such variations are considered within the scope of the present invention as defined by the claims below. While kits, compositions and methods are described in terms of "comprising," "containing," or "including" various components or steps, the kits, compositions and methods also can "consist essentially of' or "consist of' the various components and steps. Whenever a numerical range with a lower limit and an upper limit is disclosed, any number and any included range falling within the range is specifically disclosed. In particular, every range of values (of the form, "from about a to about b," or, equivalently, "from approximately a to b") disclosed herein is to be understood to set forth every number and range encompassed within the broader range of values. Also, the terms in the claims have their plain, ordinary meaning unless otherwise explicitly and clearly defined by the patentee. Moreover, the indefinite articles "a" or "an", as used in the claims, are defined herein to mean one or more than one of the element that it introduces.

## Claims

1. A system for treating or cleansing an ocular region, the system consisting essentially of:
(A) a tubular applicator (110) having a first end (110a) and an opposed second end (110b), the first end having an opening, wherein the tubular applicator consists of:
(i) a first chamber (120a);
(ii) a second chamber (120b), wherein the first chamber is adjacent the first end of the applicator and the second chamber is adjacent the second end of the of the applicator;
(iii) a rupturable sealable element (140) disposed within an internal cavity of the tubular applicator, the sealable element defining the first chamber and the second chamber within the internal cavity; and
(iv) a self-saturating dispenser (130) for the ocular composition, wherein the dispenser is bonded to an external surface of a first end of the applicator, wherein the dispenser envelopes the opening at the first end of the applicator, wherein the dispenser comprises an absorbent material selected from the group consisting of foam, sponge, fiber, felt, cotton, rayon, synthetic foam, synthetic sponge, textile and synthetic fiber; and
(B) an ocular composition, wherein the second chamber is pre-filled with the ocular composition, wherein rupturing of the sealable element creates a passage between the first and second chambers for the ophthalmic composition to flow from the second chamber to the first chamber and through the opening at the first end, wherein the dispenser is configured to receive a desired amount of the ocular composition through the opening.

2. The system according to Claim 1, wherein the ocular composition comprises:
a surfactant mixture comprising PEG-80 sorbitan laurate, sodium trideceth sulphate, PEG-150 distearate, cocamidopropylhydroxy sultaine, lauroamphocarboxy glycinate, and sodium laureth-13 carboxylate, the surfactant mixture present in a concentration of 7-10%;
PEG-15 tallow polyamine present in a concentration of 0.1-0.5%;
sodium chloride present in a concentration of 0.6-0.9%, at least one microbiological preservative selected from the group consisting of Quaternium-15 and benzyl alcohol present in concentration of 0.1-0.5% and
a chelating agent present in a concentration of 0-0.1 %.

3. The system according to Claim 1, wherein the ocular composition comprises:
PHMB;
1,2-hexanediol, and
1,2-octanediol; and
a pH stabilizing surfactant solution.

4. The system according to Claim 1, wherein the ocular composition comprises:
about 45% to about 55% tea tree oil;
about 15% to about 25% sea buckthorn oil; and
about 25% to about 35% caprylic capric triglyceride.

5. The system according to Claim 1, wherein the applicator (110) comprises a material selected from a group consisting of plastic, polymer, resin, synthetic polymer and composite material.

6. The system according to Claim 1, wherein upon breaking the sealable element (140), the ocular composition is capable of flowing from the second chamber (120b) to the first chamber (120a).

7. A kit (200) for debridement of Demodex, the kit comprising an enclosure (230) for housing:
(i) at least a first individually wrapped package (210) containing the system according to Claim 1, wherein the ocular composition comprises a first ocular composition, the first ocular composition comprising polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate;
(ii) at least a second individually wrapped package (210) containing the system according to Claim 1, wherein the ocular composition comprises a second ocular composition, the second ocular composition comprising a mixture of tea tree oil, sea buckthorn oil and a medium chain triglyceride;
(iii) at least one of a double-ended soft brush or a pair of tweezers for removing debris from the eyelids and eyelashes; and
(iv) one or more sterile saline wipes.

8. An ocular composition for use in a method for treating Demodex comprising:
(i) providing the kit (200) according to Claim 7;
(ii) holding the first individually wrapped package (210) such that the dispenser end is pointed downward and pressing firmly on a tip of the applicator that is opposite the dispenser to rupture the sealable element thereby enabling the first ocular composition to saturate the dispenser;
(iii) cleansing a surface of the eyebrows, closed eyelids and eyelashes by dispensing the first ocular composition through the dispenser;
(iv) removing any debris from the area to be treated using either the brush or tweezers;
(v) removing the first ocular composition by rinsing the cleansed surface;
(vi) holding the second individually wrapped package such that the dispenser end is pointed downward and pressing firmly on a tip of the applicator that is opposite the dispenser to rupture the sealable element thereby enabling the second ocular composition to saturate the dispenser; and
(vii) treating the cleansed surface by gently applying the second ocular composition to the cleansed surface.

9. The ocular composition for use according to Claim 8, further comprising removing any additional debris from treated surface.

10. The ocular composition for use according to Claim 8, further comprising removing the second ocular composition.

11. The ocular composition for use according to Claim 10, further comprising repeating steps (i) - (iv), wherein the first ocular composition is allowed to remain on the cleansed surface.

12. A kit (200) for meibomian gland expression comprising an enclosure (230) for housing:
(i) the system (210) according to Claim 1, wherein the ocular composition comprises polyhexamethylene biguanide, 1,2-hexanediol, 1,2-octanediol, D-panthenol, cocoamphodiacetate disodium, polyoxyethylene-80 sorbitan monolaurate, decyl polyglucoside, methyl gluceth-20, and PEG-120 methyl glucose dioleate;
(ii) a pair of moist heat therapy goggles for combining moisture and heat to open up clogged meibomian glands by stimulating tear production and preventing evaporation of tears;
(iii) one or more anesthetic gels;
(iv) a paddle for expressing meibum from the meibomian glands;
(v) a double-ended brush for removing debris from the eyelids and eyelashes;
(vi) an eye wash for irrigating or flushing foreign bodies and debris from the eye;
(vii) an eyelid cleanser for removing oil, debris and pollen from the eyelids;
(viii) an eyelid spray containing vitamins A, C and E; and
(ix) an ophthalmic emulsion to stabilize tear film and protect against moisture loss.

13. An ocular composition for use in a method for meibomian gland expression comprising:
(i) providing the kit (200) according to Claim 12;
(ii) instructing a patient to wear the moist heat therapy goggles for at least around 10-15 minutes in order to open up the clogged meibomian glands;
(iii) applying the anesthetic gel to the eyes;
(iv) after about 4-10 minutes, expressing the meibomian glands with the paddle;
(v) cleansing closed eyelids to remove excessive sebum/oil from the eyelids by dispensing the ocular composition through the dispenser;
(vi) removing debris from the cleansed eyelids using the brush; and
(vii) irrigating out the eye using the eye wash.

## Patentansprüche

1. System zum Behandeln oder Reinigen einer Augenregion, wobei das System im Wesentlichen aus Folgendem besteht:
(A) einem rohrförmigen Applikator (110) mit einem ersten Ende (110a) und einem gegenüberliegenden zweiten Ende (110b), wobei das erste Ende eine Öffnung aufweist, wobei der rohrförmige Applikator aus Folgendem besteht:
(i) einer ersten Kammer (120a);
(ii) einer zweiten Kammer (120b), wobei die erste Kammer dem ersten Ende des Applikators benachbart ist und die zweite Kammer dem zweiten Ende des Applikators benachbart ist;
(iii) einem zerreißbaren dicht verschließbaren Element (140), das innerhalb eines inneren Hohlraums des rohrförmigen Applikators angeordnet ist, wobei das dicht verschließbare Element die erste Kammer und die zweite Kammer innerhalb des inneren Hohlraums definiert; und
(iv) einem selbstsättigenden Spender (130) für die Augenzusammensetzung, wobei der Spender mit einer Außenfläche eines ersten Endes des Applikators verbunden ist, wobei der Spender die Öffnung an dem ersten Ende des Applikators umhüllt, wobei der Spender ein saugfähiges Material, ausgewählt aus der Gruppe bestehend aus Schaumstoff, Schwamm, Faser, Filz, Baumwolle, Rayon, synthetischem Schaumstoff, synthetischem Schwamm, Textil und synthetischer Faser, umfasst; und
(B) einer Augenzusammensetzung, wobei die zweite Kammer mit der Augenzusammensetzung vorgefüllt ist, wobei das Zerreißen des dicht verschließbaren Elements einen Durchlass zwischen der ersten und zweiten Kammer schafft, damit die ophthalmische Zusammensetzung aus der zweiten Kammer in die erste Kammer und durch die Öffnung an dem ersten Ende fließt, wobei der Spender dazu konfiguriert ist, eine gewünschte Menge der Augenzusammensetzung durch die Öffnung aufzunehmen.

2. System nach Anspruch 1, wobei die Augenzusammensetzung Folgendes umfasst:
ein Tensidgemisch, umfassend PEG-80 Sorbitanlaurat, Natriumtridecethsulphat, PEG-150 Distearat, Cocamidopropyl-Hydroxysultain, Lauroamphocarboxyglycinat und Natriumlaureth-13 Carboxylat, wobei das Tensidgemisch in einer Konzentration von 7-10 % vorliegt;
PEG-15 Talg-Polyamin, das in einer Konzentration von 0,1-0,5 % vorliegt;
Natriumchlorid, das in einer Konzentration von 0,6-0,9 % vorliegt, mindestens ein mikrobiologisches Konservierungsmittel, ausgewählt aus der Gruppe bestehend aus Quaternium-15 und Benzylalkohol, das in einer Konzentration von 0,1-0,5 % vorliegt, und einen Chelatbildner, der in einer Konzentration von 0-0,1 % vorliegt.

3. System nach Anspruch 1, wobei die Augenzusammensetzung Folgendes umfasst:
PHMB;
1,2-Hexandiol und
1,2-Octandiol; und
eine pH-stabilisierende Tensidlösung.

4. System nach Anspruch 1, wobei die Augenzusammensetzung Folgendes umfasst:
etwa 45 % bis etwa 55 % Teebaumöl;
etwa 15 % bis etwa 25 % Sanddornöl; und
etwa 25 % bis etwa 35 % Capryl-/Caprin-Triglycerid.

5. System nach Anspruch 1, wobei der Applikator (110) ein Material, ausgewählt aus einer Gruppe bestehend aus Kunststoff, Polymer, Harz, synthetischem Polymer und Verbundwerkstoff, umfasst.

6. System nach Anspruch 1, wobei beim Brechen des dicht verschließbaren Elements (140) die Augenzusammensetzung in der Lage ist, aus der zweiten Kammer (120b) in die erste Kammer (120a) zu fließen.

7. Kit (200) zum Debridement von Demodex, wobei das Kit eine Hülle (230) umfasst, um Folgendes aufzunehmen:
(i) mindestens ein erstes einzeln verpacktes Päckchen (210), welches das System nach Anspruch 1 enthält, wobei die Augenzusammensetzung eine erste Augenzusammensetzung umfasst, wobei die erste Augenzusammensetzung Polyhexamethylenbiguanid, 1,2-Hexandiol, 1,2-Octandiol, D-Panthenol, Cocoamphodiacetat-Disodium, Polyoxyethylen-80 Sorbitan Monolaurat, Decyl-Polyglucosid, Methylgluceth-20 und PEG-120 Methylglucose Dioleat umfasst;
(ii) mindestens ein zweites einzeln verpacktes Päckchen (210), welches das System nach Anspruch 1 enthält, wobei die Augenzusammensetzung eine zweite Augenzusammensetzung umfasst, wobei die zweite Augenzusammensetzung ein Gemisch aus Teebaumöl, Sanddornöl und einem mittelkettigen Triglycerid umfasst;
(iii) mindestens eines aus einer doppelendigen weichen Bürste oder einer Pinzette zum Entfernen von Schmutz von den Augenlidern und Wimpern; und
(iv) ein oder mehrere mit steriler Kochsalzlösung getränkte Tücher.

8. Augenzusammensetzung zur Verwendung in einem Verfahren zum Behandeln von Demodex, das Folgendes umfasst:
(i) Bereitstellen des Kits (200) nach Anspruch 7;
(ii) Halten des ersten einzeln verpackten Päckchens (210) derart, dass das Spenderende nach unten zeigt, und festes Drücken auf eine Spitze des Applikators, die dem Spender gegenüberliegt, um das dicht verschließbare Element zu zerreißen, wodurch die erste Augenzusammensetzung in die Lage versetzt wird, den Spender zu sättigen;
(iii) Reinigen einer Oberfläche der Augenbrauen, der geschlossenen Augenlider und Wimpern durch Abgeben der ersten Augenzusammensetzung durch den Spender;
(iv) Entfernen von Schmutz aus dem zu behandelnden Bereich unter Verwendung der Bürste oder der Pinzette;
(v) Entfernen der ersten Augenzusammensetzung durch Abspülen der gereinigten Oberfläche;
(vi) Halten des zweiten einzeln verpackten Päckchens derart, dass das Spenderende nach unten zeigt, und festes Drücken auf eine Spitze des Applikators, die dem Spender gegenüberliegt, um das dicht verschließbare Element zu zerreißen, wodurch die zweite Augenzusammensetzung in die Lage versetzt wird, den Spender zu sättigen; und
(vii) Behandeln der gereinigten Oberfläche durch vorsichtiges Auftragen der zweiten Augenzusammensetzung auf die gereinigte Oberfläche.

9. Augenzusammensetzung zur Verwendung nach Anspruch 8, ferner ein Entfernen von weiterem Schmutz von der behandelten Oberfläche umfassend.

10. Augenzusammensetzung zur Verwendung nach Anspruch 8, ferner ein Entfernen der zweiten Augenzusammensetzung umfassend.

11. Augenzusammensetzung zur Verwendung nach Anspruch 10, ferner ein Wiederholen der Schritte (i) - (iv) umfassend, wobei die erste Augenzusammensetzung auf der gereinigten Oberfläche verbleiben darf.

12. Kit (200) zum Ausdrücken der Meibom-Drüsen, das eine Hülle (230) umfasst, um Folgendes aufzunehmen:
(i) das System (210) nach Anspruch 1, wobei die Augenzusammensetzung Polyhexamethylenbiguanid, 1,2-Hexandiol, 1,2-Octandiol, D-Panthenol, Cocoamphodiacetat-Disodium, Polyoxyethylen-80 Sorbitan-Monolaurat, Decyl-Polyglucosid, Methylgluceth-20 und PEG-120 Methylglucose-Dioleat umfasst;
(ii) eine Feuchtwärmetherapiebrille zum Kombinieren von Feuchtigkeit und Wärme, um verstopfte Meibom-Drüsen zu öffnen, indem die Tränenproduktion angeregt und die Verdunstung von Tränen verhindert wird;
(iii) ein oder mehrere Narkosegele;
(iv) einen Spatel zum Ausdrücken von Meibom-Sekret aus den Meibom-Drüsen;
(v) eine doppelendige Bürste zum Entfernen von Schmutz von den Augenlidern und Wimpern;
(vi) eine Augenspülung zum Spülen oder Wegspülen von Fremdkörpern und Schmutz aus dem Auge;
(vii) einen Augenlidreiniger zum Entfernen von Öl, Schmutz und Pollen von den Augenlidern;
(viii) ein Augenlidspray, das die Vitamine A, C und E enthält; und
(ix) eine ophthalmische Emulsion zum Stabilisieren des Tränenfilms und Schützen vor Feuchtigkeitsverlust.

13. Augenzusammensetzung zur Verwendung in einem Verfahren zum Ausdrücken der Meibom-Drüsen, das Folgendes umfasst:
(i) Bereitstellen des Kits (200) nach Anspruch 12;
(ii) Anweisen eines Patienten, die Feuchtwärmetherapiebrille mindestens etwa 10-15 Minuten lang zu tragen, um die verstopften Meibom-Drüsen zu öffnen;
(iii) Auftragen des Narkosegels auf die Augen;
(iv) nach etwa 4-10 Minuten, Ausdrücken der Meibom-Drüsen mit dem Spatel;
(v) Reinigen der geschlossenen Augenlider, um überschüssigen/s Talg/Öl von den Augenlidern zu entfernen, indem die Augenzusammensetzung durch den Spender abgegeben wird;
(vi) Entfernen von Schmutz von den gereinigten Augenlidern unter Verwendung der Bürste; und
(vii) Ausspülen des Auges unter Verwendung der Augenspülung.

## Revendications

1. Système de traitement ou de nettoyage d'une région oculaire, le système étant essentiellement constitué :
(A) d'un applicateur tubulaire (110) ayant une première extrémité (110a) et une seconde extrémité opposée (110b), la première extrémité ayant une ouverture, l'applicateur tubulaire étant constitué :
(i) d'une première chambre (120a) ;
(ii) d'une seconde chambre (120b), la première chambre étant adjacente à la première extrémité de l'applicateur, et la seconde chambre étant adjacente à la seconde extrémité de l'applicateur ;
(iii) d'un élément cassable pouvant être scellé (140) disposé dans une cavité interne de l'applicateur tubulaire, l'élément pouvant être scellé définissant la première chambre et la seconde chambre à l'intérieur de la cavité interne ; et
(iv) d'un distributeur d'auto-saturation (130) pour la composition oculaire, le distributeur étant relié à une surface externe d'une première extrémité de l'applicateur, le distributeur enveloppant l'ouverture au niveau de la première extrémité de l'applicateur, le distributeur comprenant un matériau absorbant choisi dans le groupe constitué d'une mousse, d'une éponge, d'une fibre, d'un feutre, d'un coton, d'une rayonne, d'une mousse synthétique, d'une éponge synthétique, d'un textile et d'une fibre synthétique ; et
(B) d'une composition oculaire, la seconde chambre étant pré-remplie avec la composition oculaire, le bris de l'élément pouvant être scellé créant un passage entre les première et seconde chambres pour permettre l'écoulement de la composition ophtalmique de la seconde chambre à la première chambre et à travers l'ouverture au niveau de la première extrémité, le distributeur étant configuré pour recevoir une quantité souhaitée de la composition oculaire à travers l'ouverture.

2. Système selon la revendication 1, dans lequel la composition oculaire comprend :
un mélange de tensioactifs comprenant du laurate de sorbitane PEG-80, du sodium-tridéceth-sulfate, du distéarate PEG-150, du cocamidopropyl hydroxysultaïne, du glycinate de lauroamphocarboxyle et du carboxylate de laureth-13 sodique, le mélange de tensioactifs étant présent à une concentration comprise entre 7 et 10 % ;
la polyamine de suif PEG-15 étant présente à une concentration comprise entre 0,1 et 0,5 % ;
le chlorure de sodium étant présent à une concentration comprise entre 0,6 et 0,9 %, au moins un conservateur microbiologique choisi dans le groupe constitué du quatemium-15 et de l'alcool benzylique étant présent à une concentration comprise entre 0,1 et 0,5 %, et un agent chélatant étant présent à une concentration comprise entre 0 et 0,1 %.

3. Système selon la revendication 1, dans lequel la composition oculaire comprend :
du PHMB ;
du 1,2-hexanediol ;
du 1,2-octanediol ; et
une solution de tensioactive de stabilisation de pH.

4. Système selon la revendication 1, dans lequel la composition oculaire comprend :
environ 45 % à environ 55 % d'huile de théier ;
environ 15 % à environ 25 % d'huile d'argousier ; et
environ 25 % à environ 35 % de triglycérides capryliques/capriques.

5. Système selon la revendication 1, dans lequel l'applicateur (110) comprend un matériau choisi dans un groupe constitué d'un plastique, d'un polymère, d'une résine, d'un polymère synthétique et d'un matériau composite.

6. Système selon la revendication 1, dans lequel, lors du bris de l'élément pouvant être scellé (140), la composition oculaire est capable de s'écouler de la seconde chambre (120b) à la première chambre (120a).

7. Kit (200) pour le débridement de Demodex, le kit comprenant un boîtier (230) conçu pour contenir :
(i) au moins un premier paquet emballé individuellement (210) contenant le système selon la revendication 1, la composition oculaire comprenant une première composition oculaire, la première composition oculaire comprenant du polyhexaméthylène biguanide, du 1,2-hexanediol, du 1,2-octanediol, du D-panthénol, du cocoamphodiacétate disodique, du monolaurate de sorbitane de polyoxyéthylène-80, du décylpolyglucoside, du méthyl-gluceth-20 et du glucosedioléate de méthyle PEG-120 ;
(ii) au moins un second paquet emballé individuellement (210) contenant le système selon la revendication 1, la composition oculaire comprenant une seconde composition oculaire, la seconde composition oculaire comprenant un mélange d'huile de théier, d'huile d'argousier et d'un triglycéride à chaîne moyenne ;
(iii) au moins l'un d'une brosse douce à double extrémité et d'une pince à épiler servant à éliminer les débris des paupières et des cils ; et
(iv) une ou plusieurs lingettes à solution saline stérile.

8. Composition oculaire destinée à être utilisée dans un procédé de traitement de Demodex, comprenant :
(i) la fourniture du kit (200) selon la revendication 7 ;
(ii) le maintien du premier paquet emballé individuellement (210) de sorte que l'extrémité de distribution est dirigée vers le bas, et le fait d'appuyer fermement sur une pointe de l'applicateur en face du distributeur afin de briser l'élément pouvant être scellé, permettant ainsi à la première composition oculaire de saturer le distributeur ;
(iii) le nettoyage d'une surface des sourcils, des paupières fermées et des cils en distribuant la première composition oculaire à travers le distributeur ;
(iv) l'élimination de tout débris de la zone à traiter en utilisant soit la brosse soit la pince à épiler ;
(v) l'élimination de la première composition oculaire en rinçant la surface nettoyée ;
(vi) le maintien du second paquet emballé individuellement de sorte que l'extrémité de distribution est dirigée vers le bas, et le fait d'appuyer fermement sur une pointe de l'applicateur en face du distributeur afin de briser l'élément pouvant être scellé, permettant ainsi à la seconde composition oculaire de saturer le distributeur ; et
(vii) le traitement de la surface nettoyée par l'application en douceur de la seconde composition oculaire sur la surface nettoyée.

9. Composition oculaire destinée à être utilisée selon la revendication 8, comprenant en outre l'élimination de tout débris supplémentaire de la surface traitée.

10. Composition oculaire destinée à être utilisée selon la revendication 8, comprenant en outre l'élimination de la seconde composition oculaire.

11. Composition oculaire destinée à être utilisée selon la revendication 10, comprenant en outre la répétition des étapes (i) à (iv), la première composition oculaire étant autorisé à rester sur la surface nettoyée.

12. Kit (200) pour l'expression de glandes de Meibomius, comprenant un boîtier (230) conçu pour contenir :
(i) le système (210) selon la revendication 1, la composition oculaire comprenant du polyhexaméthylène biguanide, du 1,2-hexanediol, du 1,2-octanediol, du D-panthénol, du disodium cocoamphodiacetate, du monolaurate de polyoxyéthylène-80 de sorbitane, du décylpolyglucoside, du méthyl gluceth-20 et du méthyl glucose-dioléate de PEG-120 ;
(ii) une paire de lunettes de traitement par la chaleur humide permettant de combiner l'humidité et la chaleur pour dégager des glandes de Meibomius obstruées en stimulant la production de larmes et en empêchant l'évaporation de larmes ;
(iii) un ou plusieurs gels anesthésiques ;
(iv) une palette d'expression de meibum à partir des glandes de Meibomius ;
(v) une brosse à double extrémité servant à éliminer les débris des paupières et des cils ;
(vi) un collyre servant à l'irrigation ou l'évacuation des corps étrangers et des débris de l'œil ;
(vii) un nettoyeur de paupières servant à éliminer l'huile, les débris et le pollen des paupières ;
(viii) un spray pour paupières contenant des vitamines A, C et E ; et
(ix) une émulsion ophtalmique servant à stabiliser le film lacrymal et à assurer la protection contre la perte d'humidité.

13. Composition oculaire destinée à être utilisé dans un procédé d'expression des glandes de Meibomius, comprenant
(i) la fourniture du kit (200) selon la revendication 12 ;
(ii) le fait de demander à un patient de porter les lunettes de traitement par la chaleur humide pendant au moins environ 10 à 15 minutes afin de dégager les glandes de Meibomius obstruées ;
(iii) l'application du gel anesthésique aux yeux ;
(iv) l'expression des glandes de Meibomius avec la palette après environ 4 à 10 minutes ;
(v) le nettoyage des paupières fermées pour éliminer l'excès de sébum/d'huile des paupières en distribuant la composition oculaire à travers le distributeur ;
(vi) l'élimination des débris des paupières nettoyées à l'aide de la brosse ; et
(vii) l'irrigation de l'œil à l'aide du collyre.
